# EUROPÄISCHE PATENTSCHRIFT

(11) **EP 0 981 321 B1**
(45) Veröffentlichungstag und Bekanntmachung des Hinweises auf die Patenterteilung: **23.02.2005**
(21) Anmeldenummer: 98924267.2
(22) Anmeldetag: 02.05.1998
(51) Int. Cl.: A61K 7/13

(54) **VERFAHREN ZUR HERSTELLUNG VON HAARFÄRBEMITTELN**
METHOD FOR PRODUCING HAIR DYE PRODUCTS
PROCEDE POUR PRODUIRE DES COLORANTS CAPILLAIRES

(30) Priorität: 12.05.1997 DE 19719504
(43) Veröffentlichungstag der Anmeldung: 01.03.2000
(73) Patentinhaber: Cognis Deutschland GmbH & Co. KG, 40589 Düsseldorf (DE)
(72) Erfinder: PITFIELD, Adrian, D-64753 Brombachtal (DE); KAHRE, Jörg, D-42799 Leichlingen (DE); BUSCH, Peter, D-40699 Erkrath (DE); FÖRSTER, Thomas, D-40699 Erkrath (DE); HENSEN, Hermann, D-42781 Haan (DE); TESMANN, Holger, D-41363 Jüchen (DE); SUMSER, Markus, D-44625 Herne (DE)
(74) Vertreter: Fabry, Bernd, Dr.
(86) Internationale Anmeldenummer: PCT/EP1998/002595
(87) Internationale Veröffentlichungsnummer: WO 1998/051267

(56) Entgegenhaltungen:
- EP-A- 0 490 053
- EP-A- 0 820 758
- US-A- 5 021 066

## Beschreibung

### Gebiet der Erfindung

Die Erfindung betrifft ein Verfahren zur Herstellung von Haarfärbemitteln, bei dem man zunächst eine PIT- bzw. Mikroemulsion herstellt und in diese die Farbstoffe kalt einrührt.

### Stand der Technik

Haarfärbemittel auf Basis von Emulsionen werden ganz überwiegend nach Heißverfahren hergestellt, d.h. zur Herstellung stabiler Produkte wird bei Temperaturen oberhalb von 60, vorzugsweise oberhalb von 80°C gearbeitet. Im Anschluß werden die Emulsionen langsam abgekühlt, was zu ganz erheblichen Kesselbelegungszeiten führt. Es ist sofort klar, daß ein Verfahren, welches die Herstellung der Mittel in der Kälte erlauben würde, signifikante ökonomische Vorteile aufweisen würde. Aus den Europäischen Patentanmeldungen EP 0490053 A1 und EP 0278660 A1 sind Haarkuren in Form von Mikroemulsionen bekannt.

Demzufolge hat die Aufgabe der vorliegenden Erfindung darin bestanden, ein solches Verfahren zur Verfügung zu stellen.

### Beschreibung der Erfindung

Gegenstand der Erfindung ist ein Verfahren zur Herstellung von Haarfärbemitteln, bei dem man zunächst unter Verwendung von Ölkörpem und Emulgatoren ausgewählt aus der Gruppe, die gebildet wird von Alkyl- und/oder Alkenyloligoglykosiden, Aniontensiden, Esterquats, Polyolpoly-12-hydroxystearaten, Fettalkoholen und Fettalkoholpolyethylenglycolethem, eine wäßrige Zubereitung in Form einer PIT-Emulsion oder Mikroemulsion herstellt und in diese die Farbstoffe bzw. Kuppler- und Entwicklersubstanzen in einem Kaltprozeß im Bereich von 15-25 °C einrührt.

Überraschenderweise wurde gefunden, daß in PIT- bzw. Mikroemulsionen, die unter Verwendung ausgewählter Emulgatoren hergestellt wurden, Farbstoffe auch kalt eingearbeitet werden können, wodurch sich die Herstellzeiten signifikant verkürzen lassen. Die verwendeten PIT- bzw. Mikroemulsionen können dabei alle erforderlichen Komponenten mit Ausnahme der Farbstoffe enthalten. Sie können jedoch im einfachsten Fall auch nur aus Ölkörpern, Emulgatoren sowie gegebenenfalls weiteren temperaturbeständigen Zusatzstoffen bestehen, so daß empfindlichere Komponenten, wie beispielsweise Proteinderivate oder Parfümöle, zusammen mit den Farbstoffen anschließend kalt eingearbeitet werden.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht.

### Emulgatoren

Als Emulgatoren zur Herstellung der PIT- bzw. Mikroemulsion kommen Alkyl- und/oder Alkenyloligoglucoside, Aniontensiden, Esterquats, Polyolpoly-12-hydroxystearate, Fettsäureester, Fettalkohole und Fettalkoholpofyethylenglycolethern sowie deren Gemische in Frage.

**Alkyl- und Alkenyloligoglykoside** stellen bekannte nichtionische Tenside dar, die der Formel **(I)** folgen,

**R**^{**1**}**O-[G]**_{**p**} **(I)** (I)

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1 0301298** und **WO 90/03977** verwiesen. Die Alkylund/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)-sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Vorzugsweise werden Alkylethersulfate auf Basis von Addukten von 1 bis 10 Mol Ethylenoxid an Fettalkohole mit 8 bis 22, vorzugsweise 12 bis 18 Kohlenstoffatomen in Form ihrer Natrium- oder Magnesiumsalze eingesetzt.

Unter der Bezeichnung **Esterquats** werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE-C1 4308794** (Henkel) ist überdies ein Verfahren zur Herstellung fester Esterquats bekannt, bei dem man die Quaternierung von Triethanolaminestem in Gegenwart von geeigneten Dispergatoren, vorzugsweise Fettalkoholen, durchführt. Übersichten zu diesem Thema sind beispielsweise von R.Puchta et al. in **Tens. Surf. Det., 30, 186 (1993),** M.Brock in **Tens. Surf. Det. 30, 394 (1993),** R.Lagerman et al. in **J. Am. Oil. Chem. Soc., 71, 97 (1994)** sowie I.Shapiro in **Cosm.Toil. 109, 77 (1994)** erschienen. Die quatemierten Fettsäuretriethanolaminestersalze folgen der Formel **(II)**, 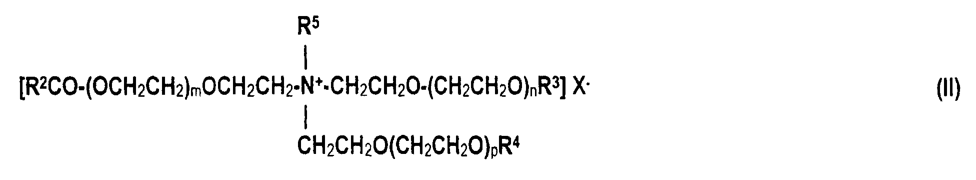 in der R²CO für einen Acylrest mit 6 bis 22 Kohlenstoffatomen, R³ und R⁴ unabhängig voneinander für Wasserstoff oder R²CO, R⁵ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quaternierten Ester können die Fettsäuren und das Triethanolamin im molaren Verhältnis von 1,1 : 1 bis 3 : 1 eingesetzt werden. lm Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (lodzahl 0 bis 40) ab. Aus anwendungstechnischer Sicht haben sich quaternierte Fettsäuretriethanolaminestersalze der Formel **(II)** als besonders vorteilhaft erwiesen, in der R²CO für einen Acylrest mit 16 bis 18 Kohlenstoffatomen, R³ für R²CO, R⁴ für Wasserstoff, R⁵ für eine Methylgruppe, m, n und p für 0 und X für Methylsulfat steht.

Neben den quaternierten Fettsäuretriethanolaminestersalzen kommen als Esterquats ferner auch quaternierte Estersalze von Fettsäuren mit Diethanolalkylaminen der Formel **(III)** in Betracht, 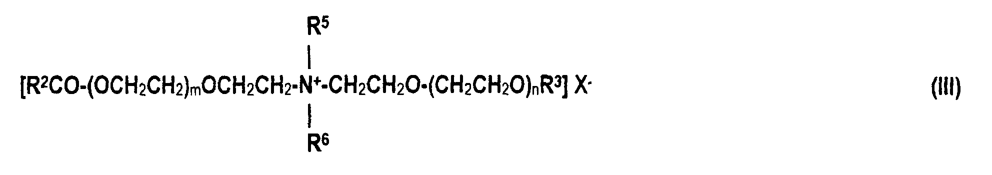 in der R²CO für einen Acylrest mit 6 bis 22 Kohlenstoffatomen, R³ für Wasserstoff oder R²CO, R⁵ und R⁶ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel **(IV)** zu nennen, 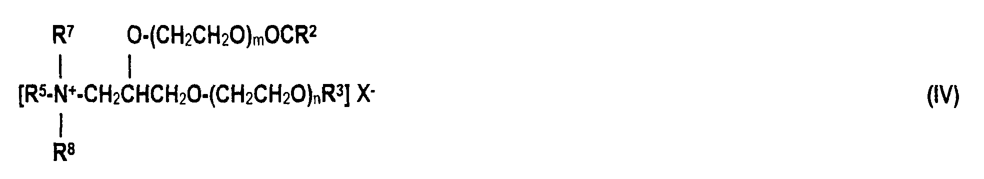 in der R²CO für einen Acylrest mit 6 bis 22 Kohlenstoffatomen, R² für Wasserstoff oder R²CO, R⁵, R⁷ und R⁸ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht. Hinsichtlich der Auswahl der bevorzugten Fettsäuren und des optimalen Veresterungsgrades gelten die für **(II)** genannten Beispiele auch für die Esterquats der Formeln **(III)** und **(IV)**.

Bei den **Polyolpoly-12-hydroxystearaten** handelt es sich um bekannte Stoffe, die beispielsweise unter den Marken "Dehymuls® PGPH" oder "Eumulgin® VL 75" (Abmischung mit Coco Glucosides im Gewichtsverhältnis 1:1) von der Henkel KGaA, Düsseldorf/FRG vertrieben werden. In diesem Zusammenhang sei ferner auf die internationale Patentanmeldung **WO 95/34528** (Henkel) verwiesen. Die Polyolkomponente der Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
(a) Glycerin und Polyglycerin;
(b) Alkylenglycole, wie beispielsweise Ethylenglycol, Diethylenglycol, Propylenglycol;
(c) Methyolverbindungen, wie insbesondere Trimethylolethan, Trimethylolpropan, Trimethylolbutan, Pentaerythrit und Dipentaerythrit;
(d) Alkyloligoglucoside mit 1 bis 22, vorzugsweise 1 bis 8 und insbesondere 1 bis 4 Kohlenstoffen im Alkylrest, wie beispielsweise Methyl- und Butylglucosid;
(e) Zuckeralkohole mit 5 bis 12 Kohlenstoffatomen, wie beispielsweise Sorbit oder Mannit,
(f) Zucker mit 5 bis 12 Kohlenstoffatomen, wie beispielsweise Glucose oder Saccharose;
(g) Aminozucker wie beispielsweise Glucamin.

Unter den erfindungsgemäß einzusetzenden Emulgatoren kommt Umsetzungsprodukten auf Basis von Polyglycerin wegen ihrer ausgezeichneten anwendungstechnischen Eigenschaften eine besondere Bedeutung zu. Als besonders vorteilhaft hat sich die Verwendung von ausgewählten Polyglycerinen erwiesen, die die folgende Homologenverteilung aufweisen (in Klammern angegeben sind die bevorzugten Bereiche):

| | | |
|---|---|---|
| Glycerin | 5 bis 35 (15 bis 30) | Gew.-% |
| Diglycerine | 15 bis 40 (20 bis 32) | Gew.-% |
| Triglycerine | 10 bis 35 (15 bis 25) | Gew.-% |
| Tetraglycerine | 5 bis 20 ( 8 bis 15) | Gew.-% |
| Pentaglycerine | 2 bis 10 (3 bis 8) | Gew.-% |
| Oligoglycerine | ad 100 | Gew.-% |

**Fettalkohole** und **Fettalkoholpolyethylenglycolether**, die des weiteren als Emulgatoren in Frage kommen, folgen vorzugsweise der Formel **(V)**,

**R**^{**9**}**O(CH**_{**2**}**CH**_{**2**}**O)**_{**n**}**H (V)** (V)

in der R⁹ für einen linearen oder verzweigten Alkyl- und/oder Alkenylrest mit 12 bis 22, vorzugsweise 16 bis 18 Kohlenstoffatomen und n für 0 oder Zahlen von 1 bis 25 steht. Typische Beispiele sind Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen und deren Addukte mit 1 bis 25, vorzugsweise 10 bis 20 Mol Ethylenoxid. Besonders bevorzugt ist der Einsatz von Cetylalkohol, Stearylalkohol, Cetearylalkohol sowie von Anlagerungsprodukten von 10 bis 20 Mol Ethylenoxid an Cetearylalkohol.

### PIT- und Mikroemulsionen

Eine bevorzugte Herstellmethode besteht darin, sogenannte **PIT-Emulsionen** herzustellen, in dem man die Ölkörper und die Emulgatoren auf die Herstelltemperatur aufheizt und mit der ebenfalls auf Herstelltemperatur aufgeheizten benötigten Menge Wasser mischt. Die Herstelltemperatur sollte oberhalb des Phaseninversionstemperaturbereichs liegen. Diesen Temperaturbereich sollte man daher zunächst anhand eines Probeansatzes ermitteln. Dabei werden alle Komponenten der Emulsion, einschließlich des Wassers erhitzt, wobei man mit Hilfe eines Leitfähigkeitsmeßgerätes den Temperaturbereich bestimmt, bei dem - infolge Phasenumkehr - die Leitfähigkeit stark abnimmt. Eine Übersicht zu PIT-Emulsionen findet sich von A.Wadle et al. in **Parf.Kosm. 72, 250 (1996);** stellvertretend für den umfangreichen Stand der Technik sei auf die Druckschriften **DE-A1 3819193, DE-A1 4010393, DE-A1 4140562, DE-A1 4318171** und **DE-A1 4337041** (Henkel) verwiesen.

Anstelle der PIT-Emulsionen können unter Verwendung der Ölkörper und ausgewählten Emulgatoren auch sogenannte **Mikroemulsionen** hergestellt werden, bei denen es sich um optisch isotrope, thermodynamisch stabile Systeme handelt, die eine wasserunlösliche Ölkomponente, Emulgatoren - vorzugsweise Alkylglucoside - und Wasser enthalten. Das klare bzw. transparente Aussehen der Mikroemulsionen ist eine Folge der geringen Teilchengröße der dispergierten Emulsionströpfchen, die im wesentlichen unter 100 nm, im Mittel immer unter 50 nm, liegt. Übersichten zu Herstellung und Anwendung von Mikroemulsionen finden sich von H.Eicke in **SÖFW-Journal, 118, 311 (1992)** und Th.Förster et al. in **SÖFW-Journal, 122, 746 (1996);** des weiteren sei auf die Druckschriften **DE-A1 4411557** (Henkel) und **EP-A1 0687206** (L'Oréal) verwiesen.

Die PIT- bzw. Mikroemulsionen können die Ölkörper in Mengen von 10 bis 90, vorzugsweise 20 bis 50 und die Emulgatoren in Mengen von 1 bis 20, vorzugsweise 5 bis 15 Gew.-% - jeweils bezogen auf die Emulsionen - enthalten.

### Farbstoffe

Im Sinne des erfindundungsgemäßen Verfahrens werden im Anschluß an die Herstellung der PIT- bzw. Mikroemulsion die Farbstoffe kalt eingerührt. Als Farbstoffe kommen dabei beispielsweise **direktziehende Farbstoffe**, z.B. aus der Gruppe der Nitrophenylendiamine, Nitroaminophenole, Anthrachinone oder Indophenole in Betracht, wie z.B. die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16, Basic Brown 17, Pikraminsäure und Rodol 9 R bekannten Verbindungen sowie 4-Amino-2-nitrodiphenylamin-2'-carbonsäure, 6-Nitro-1,2,3,4-tetrahydrochinoxalin, (N-2,3-Dihydroxypropyl-2-nitro-4-trifluormethyl)amino-benzol und 4-N-Ethyl-1,4-bis(2'-hydroxyethylamino)-2-nitrobenzol-hydrochlorid. Weiterhin können den Emulsionen auch in der Natur vorkommende Farbstoffe wie beispielsweise Henna rot, Henna neutral, Henna schwarz, Kamillenblüte, Sandelholz, schwarzer Tee, Faulbaumrinde, Salbei, Blauholz, Krappwurzel, Catechu, Sedre und Alkannawurzel zugesetzt werden.

Neben den Direktziehern können den Emulsionben auch **Oxidationsfarbstoffe**, bestehend aus Entwickler- und Kupplerkomponente zugesetzt werden. Als Entwicklerkomponenten werden beispielsweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Spezielle Vertreter sind u.a. p-Toluylendiamin, p-Aminophenol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5 und 4-Amino-3-methylphenol, 2-(2-Hydroxyethyl)-1,4-aminobenzol und 2,4,5,6-Tetraaminopyrimidin. Als Kupplerkomponenten werden in der Regel m-Phenylendiaminderivate, Naphthole, Resorcin und Resorcinderivate, Pyrazolone, m-Aminophenole sowie Pyridin-Derivate verwendet. Als Kupplersubstanzen eignen sich insbesondere 1-Naphthol, Pyrogallol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 2-Chlor-6-methyl-3-aminophenol, 2-Methylresorcin, 2,5-Dimethylresorcin, 2,6-Dihydroxypyridin und 2,6-Diaminopyridin.

Bezüglich weiterer Farbstoffkomponenten wird ausdrücklich auf die Colipa-Liste, herausgegeben vom Industrieverband Körperpflege und Waschmittel, Frankfurt, Bezug genommen. Die Farbstoffe können den Emulsionen in Mengen von 0,1 bis 10, vorzugsweise 1 bis 5 Gew.-% - bezogen auf die Emulsionen - zugesetzt werden. Unter kaltem Rühren ist in diesem Zusammenhang eine Vermischung bei Umgebungstemperatur, d.h. im Bereich von 15 bis 25°C zu verstehen.

### Hilfs- und Zusatzstoffe

Im Sinne des erfindungsgemäßen Verfahrens können die Emulsionen weitere Hilfs- und Zusatzstoffe enthalten, wie beispielsweise Co-Emulgatoren Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren oderf Parfümöle.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkyl(ether)phosphate, Alkylamidobetaine und/oder Proteinfettsäurekondensate, letztere vorzugsweise auf Basis von Weizenproteinen.

Als **Co-Emulgatoren** kommen beispielsweise nichtionogene Tenside aus mindestens einer der folgenden Gruppen in Frage:
(1) C_{12/18}-Fettsäuremono- und -diester von Anlagerungsprodukten von 1 bis 30 Mol Ethylenoxid an Glycerin;
(2) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(3) Anlagerungsprodukte von 15 bis 60 Mol Ethylenoxid an Ricinusöl und/oder gehärtetes Ricinusöl;
(4) Anlagerungsprodukte von 2 bis 15 Mol Ethylenoxid an Ricinusöl und/oder gehärtetes Ricinusöl;
(5) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(6) Trialkylphosphate sowie Mono-, Di- und/oder Tri-PEG-alkylphosphate;
(7) Wollwachsalkohole;
(8) Polysiloxan-Polyalkyl-Polyether-Copolymere bzw. entsprechende Derivate;
(9) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(10) Polyalkylenglycole.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte VinylpyrrolidonNinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentriamin (Gartaretine®/Sandoz), Copolymere der Acrylsäure mit Dimethyldiallylammoniumchlorid (Merquat® 550/Chemviron), Polyaminopolyamide wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar@ C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor-, glucosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Film bildner** sind beispielsweise Chitosan, mikrokristallines Chitosan, quaterniertes Chitosan, Polyvinylpyrrolidon, Vinyl-pyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als Quellmittel für wäßrige Phasen können kolloidale Kieselsäure, Schichsilicate wie Montmorillonit, Clay Mineralstoffe, Pemulen sowie alkylmodifizierte Carbopoltypen (Goodrich) dienen. Als **Konservierungsmittel** eignen sich beispielsweise Phenoxyethanol, Formaldehydlösung, Parabene, Pentandiol oder Sorbinsäure. Zur Einstellung des pH-Wertes eignen sich Ammoniak, Amine oder basische Aminosäuren.

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, lris, Calmus), Hölzern (Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, lonon und Methylionon in Betracht.

Hierbei ist es unkritisch, ob die Hilfs- und Zusatzstoffe schon im Rahmen der Herstellung der PIT- bzw. Mikroemulsion oder erst nachträglich zugegeben werden. Die den Farbstoff enthaltende kalt hergestellte Emulsion kann dann auf die Anwendungskonzentration verdünnt und falls erforderlich mit einer entsprechenden Wasserstoffperoxid enthaltenden Emulsion in Kontakt gebracht werden, welche ihrerseits analog den PIT- bzw. Mikroemulsionen hergestellt werden können.

### Beispiele

**Beispiel 1.** Nach dem PIT-Verfahren wurde eine Emulsion hergestellt, enthaltend 33,3 g Emulgade® CM [Cetyl Isononanoate (and) Ceteareth-20 (and) Cetearyl Alcohol (and) Glyceryl Stearate (and) Glycerin (and) Cetyl Palmitate (and) Ceteareth-12, Henkel KGaA, Düsseldorf/DE], 9 g C_{8/18}-Alkylglucosid, 9 g kolloidale Kieselsäure, 3 g Ammoniumchlorid, wäßrige Ammoniaklösung ad 100 g (pH = 10,5). In die PIT-Emulsion wurde bei 20°C jeweils 7,5 mmol einer Entwicklerkomponente (N,N'-Bis(4-aminophenyl)-piperidin und einer Kupplerkomponente (Resorcin) eingerührt. Die oxidative Entwicklung wurde anschließend mit Wasserstoffperoxid durchgeführt. Die Nuance des mit dem gefärbten Haares war dunkelblond.

**Beispiel 2.** Analog Beispiel 1 wurde eine PIT-Emulsion hergestellt, enthaltend 33,3 g Lamesoft® PW 45 [Cetyl Palmitate (and) Hydrogenated Castor Oil (and) Glyceryl Stearate (and) Beheneth-10; Henkel KGaA] 9 g C_{8/18}-Alkylglucosid, 9 g kolloidale Kieselsäure, 3 g Ammoniumchlorid, wäßrige Ammoniaklösung ad 100 g (pH = 10,5). In die PIT-Emulsion wurde bei 20°C jeweils 7,5 mmol 2,4,5,6-Tetraaminopyridin und 2,6-Bis(2-hydroxyethylamino)-toluol eingerührt. Die oxidative Entwicklung wurde wiederum mit Wasserstoffperoxid durchgeführt Die Nuance des gefärbten Haares war tiefrot.

**Beispiele 3 bis 15.** Auf Basis dieser Beispiele wurden dann folgende Haarfärbecremeemulsion kalt hergestellt (Wasser ad 100 Gew.-%):

| | |
|---|---|
| Cremebasis nach Beispiel 1 bzw. 2 | 50,0 Gew.-% |
| Entwicklerkomponente | 7,5 mmol |
| Kupplerkomponente | 7,5 mmol |
| Na₂SO₃ (Inhibitor) | 1,0 Gew.-% |
| (NH₄)₂SO₄ | 1,0 Gew.-% |
| konz. Ammoniaklösung | ad pH 10 |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt. Die oxidative Entwicklung der Färbung wurde mit Wasserstoffperoxidlösung als Oxidationslösung durchgeführt. Hierzu wurden 100 g der Emulsion mit 50 g Wasserstoffperoxidlösung (1, 3 oder 9 %ig) versetzt und vermischt. Die Färbecreme wurde auf ca. 5 cm lange Strähnen standardisierten, zu 90 % ergrauten, aber nicht besonders vorbehandelten Menschenhaars aufgetragen und dort 30 Minuten bei 32 °C belassen. Nach Beendigung des Färbeprozesses wurde das Haar gespült, mit einem üblichen Haarwaschmittel gewaschen und anschließend getrocknet. Es wurden Ausfärbungen gemäß Tabelle 1 gefunden:

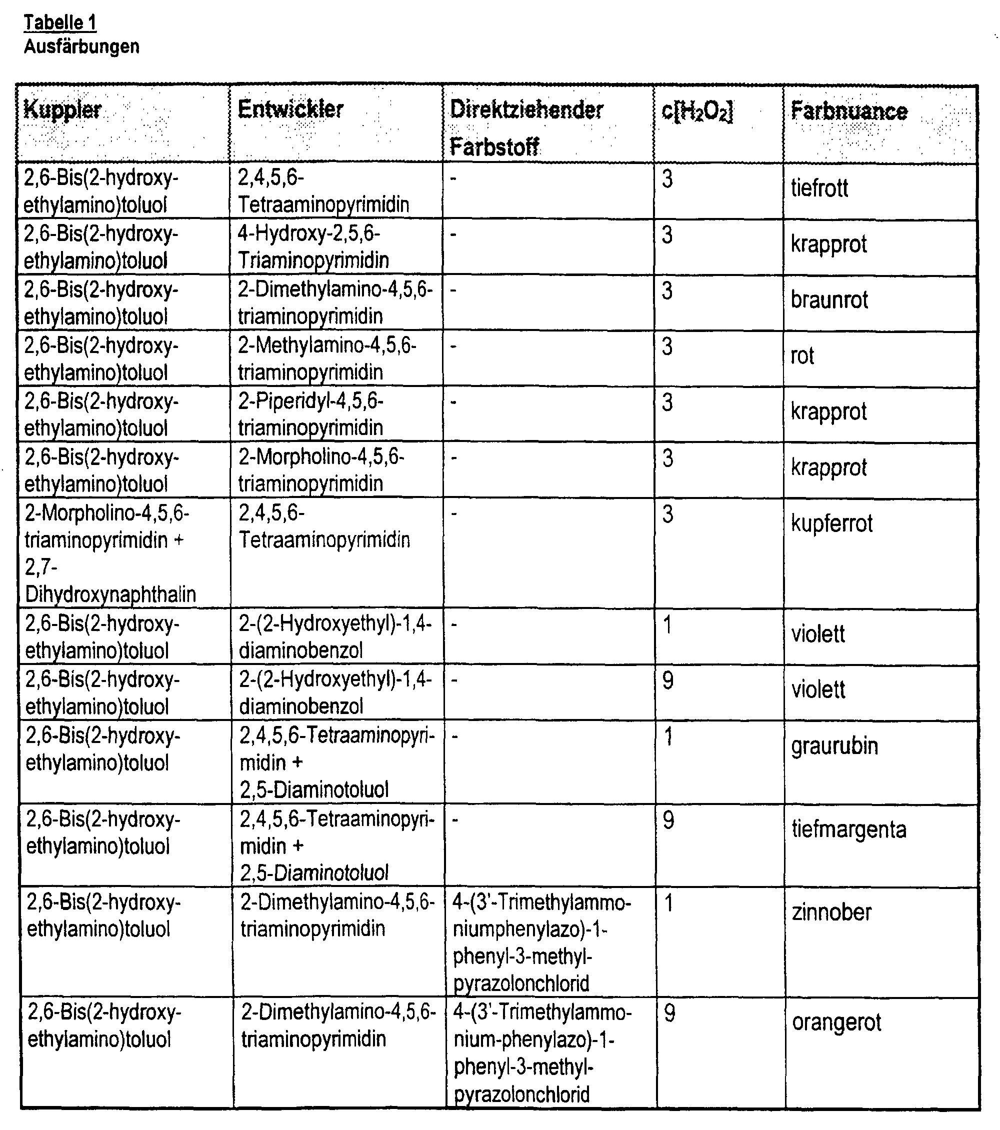

## Patentansprüche

1. Verfahren zur Herstellung von Haarfärbemitteln, bei dem man zunächst unter Verwendung von Ölkörpem und Emulgatoren ausgewählt aus der Gruppe, die gebildet wird von Alkyl- und/oder Alkenyloligoglykosiden, Aniontensiden, Esterquats, Polyolpoly-12-hydroxystearaten, Fettalkoholen und Fettalkoholpolyethylenglykolethern, eine wäßrige Zubereitung in Form einer PIT-Emulsion oder Mikroemulsion herstellt und in diese die Farbstoffe bzw. Kuppler- und Entwicklersubstanzen in einem Kaltprozeß im Bereich von 15 bis 25 °C einrührt.

2. Verfahren nach Anspruch 1, **dadurch gekennzeichnet, daß** man Ölkörper einsetzt, die ausgewählt sind aus der Gruppe, die gebildet wird von Guerbetalkoholen auf Basis von Fettalkoholen mit 6 bis 18 Kohlenstoffatomen, Estern von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estern von linearen C₆-C₂₂-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estern von C₆-C₂₂-Fettalkoholen und/oder Guerbetalkoholen mit aromatischen Carbonsäuren, pflanzlichen Ölen, verzweigten primären Alkoholen, substituierten Cyclohexanen, linearen C₆-C₂₂-Fettalkoholcarbonaten, Guerbetcarbonaten, Estern der Benzoesäure mit linearen und/oder verzweigten C₆-C₂₂-Alkoholen, Dialkylethern, Ringöffnungsprodukten von epoxidierten Fettsäureestern mit Polyolen, Siliconölen und aliphatischen bzw. naphthenischen Kohlenwasserstoffen.

3. Verfahren nach den Ansprüchen 1 und 2, **dadurch gekennzeichnet, daß** man Alkyl- und Alkenyloligoglykoside der Formel (I) einsetzt,
**R**^{**1**}**O-[G]**_{**p**} **(I)** (I)
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

4. Verfahren nach den Ansprüchen 1 bis 3, **dadurch gekennzeichnet, daß** man Alkylethersulfate einsetzt.

5. Verfahren nach den Ansprüchen 1 bis 4, **dadurch gekennzeichnet, daß** man Esterquats der Formel **(II)** einsetzt, 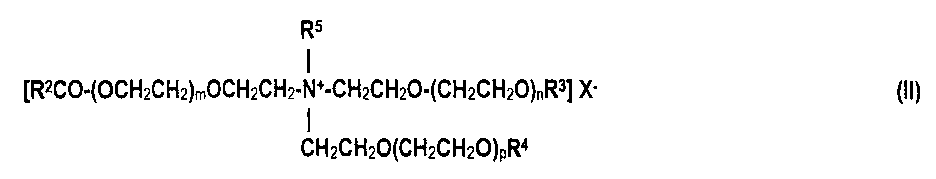 in der R²CO für einen Acylrest mit 6 bis 22 Kohlenstoffatomen, R³ und R⁴ unabhängig voneinander für Wasserstoff oder R²CO, R⁵ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

6. Verfahren nach den Ansprüchen 1 bis 4, **dadurch gekennzeichnet, daß** man Esterquats der Formel **(III)** einsetzt, 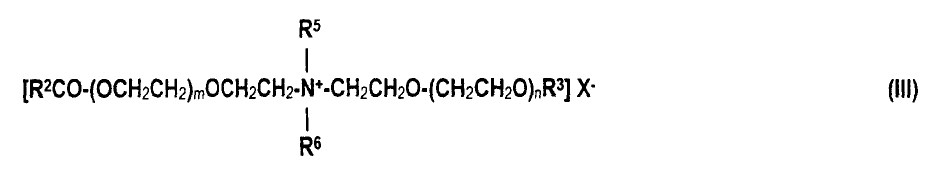 in der R²CO für einen Acylrest mit 6 bis 22 Kohlenstoffatomen, R³ für Wasserstoff oder R²CO, R⁵ und R⁶ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

7. Verfahren nach den Ansprüchen 1 bis 4, **dadurch gekennzeichnet, daß** man Esterquats der Formel **(IV)** einsetzt, 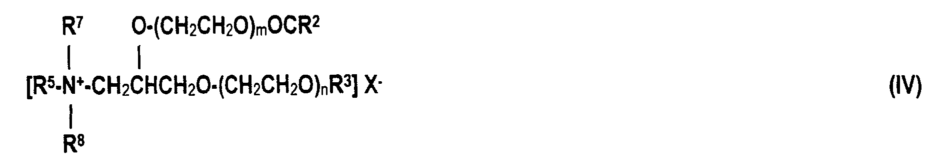 in der R²CO für einen Acylrest mit 6 bis 22 Kohlenstoffatomen, R² für Wasserstoff oder R²CO, R⁵, R⁷ und R⁸ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

8. Verfahren nach den Ansprüchen 1 bis 7, **dadurch gekennzeichnet, daß** man als Polyolpoly-12-hydroxystearate Polyglycerinpoly-12-hydroxystearate einsetzt.

9. Verfahren nach den Ansprüchen 1 bis 8, **dadurch gekennzeichnet, daß** man Fettalkohole und/oder Fettalkoholpolyethylenglycolether der Formel (II) einsetzt,
**R**^{**9**}**O(CH**_{**2**}**CH**_{**2**}**O)**_{**n**}**H (V)** (V)
in der R⁹ für einen linearen oder verzweigten Alkyl- und/oder Alkenylrest mit 12 bis 22 Kohlenstoffatomen und n für 0 oder Zahlen von 1 bis 25 steht.

10. Verfahren nach den Ansprüchen 1 bis 9, **dadurch gekennzeichnet, daß** man den Emulsionen direktziehende Farbstoffe und/oder Oxidationsfarbstoffe zusetzt.

## Claims

1. A process for the production of hair colorants in which an aqueous preparation in the form of a PIT emulsion or microemulsion is initially prepared using oil components and emulsifiers selected from the group consisting of alkyl and/or alkenyl oligoglycosides, anionic surfactants, esterquats, polyolpoly-12-hydroxystearates, fatty alcohols and fatty alcohol polyethylene glycol ethers and the dyes or rather primary and secondary intermediates are then stirred into the PIT emulsion or microemulsion in a cold process at 15 to 25°C..

2. A process as claimed in claim 1, **characterized in that** the oil components used are selected from the group consisting of Guerbet alcohols based on fatty alcohols containing 6 to 18 carbon atoms, esters of linear C₆₋₂₂ fatty acids with linear C₆₋₂₂ fatty alcohols, esters of branched C_{6B13} carboxylic acids with linear C₆₋₂₂ fatty alcohols, esters of linear C₆₋₂₂ fatty acids with branched alcohols, esters of linear and/or branched fatty acids with polyhydric alcohols and/or Guerbet alcohols, triglycerides based on C₆₋₁₀ fatty acids, liquid mono-/di-/triglyceride mixtures based on C₆₋₁₈ fatty acids, esters of C₆₋₂₂ fatty alcohols and/or Guerbet alcohols with aromatic carboxylic acids, vegetable oils, branched primary alcohols, substituted cyclohexanes, linear C₆₋₂₂ fatty alcohol carbonates, Guerbet carbonates, esters of benzoic acid with linear and/or branched C₆₋₂₂ alcohols, dialkyl ethers, ring-opening products of epoxidized fatty acid esters with polyols, silicone oils and aliphatic or naphthenic hydrocarbons.

3. A process as claimed in claims 1 and 2, **characterized in that** alkyl and alkenyl oligoglycosides corresponding to formula (I):
**R**^{**1**}**O-[G]**_{**p**} **(I)**
in which R¹ is an alkyl and/or alkenyl radical containing 4 to 22 carbon atoms, G is a sugar unit containing 5 or 6 carbon atoms and p is a number of 1 to 10,
are used

4. A process as claimed in claims 1 to 3, **characterized in that** alkyl ether sulfates are used.

5. A process as claimed in claims 1 to 4, **characterized in that** esterquats corresponding to formula (II): 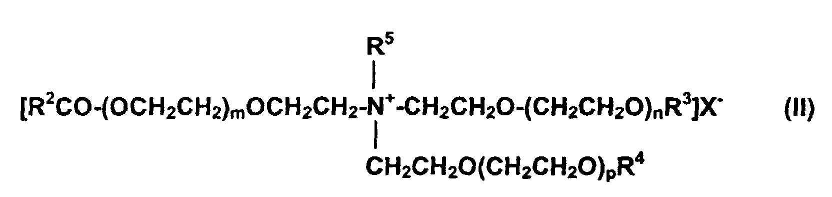 in which R²CO is an acyl group containing 6 to 22 carbon atoms, R³ and R⁴ independently of one another represent hydrogen or have the same meaning as R²CO, R⁵ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate, are used.

6. A process as claimed in claims 1 to 4, **characterized in that** esterquats corresponding to formula **(III):** 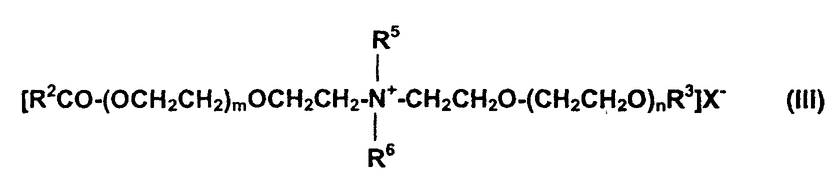 in which R²CO is an acyl group containing 6 to 22 carbon atoms, R³ is hydrogen or has the same meaning as R²CO, R⁵ and R⁶ independently of one another are alkyl groups containing 1 to 4 carbon atoms, m and n together stand for 0 or numbers of 1 to 12 and X stands for halide, alkyl sulfate or alkyl phosphate, are used.

7. A process as claimed in claims 1 to 4, **characterized in that** esterquats corresponding to formula **(VI):** 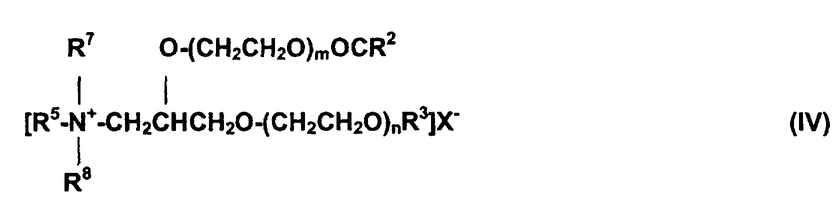 in which R²CO is an acyl group containing 6 to 22 carbon atoms, R² is hydrogen or has the same meaning as R²CO, R⁵, R⁷ and R⁸ independently of one another are alkyl groups containing 1 to 4 carbon atoms, m and n together stand for 0 or numbers of 1 to 12 and X stands for halide, alkyl sulfate or alkyl phosphate, are used.

8. A process as claimed in claims 1 to 7, **characterized in that** polyglycerol poty-12-hydroxystgearates are used as the polyolpoly-12-hydroxystearates.

9. A process as claimed in claims 1 to 8, **characterized in that** fatty alcohols and/or fatty alcohol polyethylene glycol ethers corresponding to formula **(VI):**
**R**^{**9**}**O(CH**_{**2**}**CH**_{**2**}**O)**_{**n**}**H (V)**
in which R⁹ is a linear or branched alkyl and/or alkenyl group containing 12 to 22 and n is 0 or a number of 1 to 25, are used.

10. A process as claimed in claims 1 to 9, **characterized in that** substantive dyes and/or oxidation dyes are added to the emulsions.

## Revendications

1. Procédé de préparation de colorants pour cheveux,
selon lequel
on produit tout d'abord une préparation aqueuse sous la forme d'une microémulsion ou d'une émulsion PIT, et en utilisant des corps huileux et des émulsifiants choisis dans le groupe formé par les alkyl- et/ou alcényl-oligoglycosides, les tensioactifs anioniques, les esters d'ammonium quaternaire, les poly-12-hydroxystéarates de polyols, les alcools gras et les polyéthylèneglycoléthers d'alcools gras,
on délaye les colorants ou les substances de couplage et de développement dans cette préparation selon un processus à froid qui se déroule dans un intervalle allant de 15 à 25°C.

2. Procédé selon la revendication 1,
**caractérisé en ce qu'**
on utilise des corps huileux choisis dans le groupe formé par les alcools de Guerbet à base d'alcools gras comportant de 6 à 18 atomes de carbone, les esters d'acides gras linéaires en C₆ à C₂₂ et d'alcools gras linéaires en C₆ à C₂₂, les esters d'acides carboxyliques ramifiés en C₆ à C₁₃ et d'alcools gras linéaires en C₆ à C₂₂, les esters d'acides gras linéaires en C₆ à C₂₂ et d'alcools ramifiés, les esters d'acides gras linéaires et/ou ramifiés et d'alcools polyvalents et/ou d'alcools de Guerbet, les triglycérides à base d'acides gras en C₆ à C₁₀, les mélanges de mono-/di-/triglycérides liquides à base d'acides gras en C₆ à C₁₈, les esters d'alcools gras en C₆ à C₂₂ et/ou d'alcools de Guerbet et d'acides carboxyliques aromatiques, les huiles végétales, les alcools primaires ramifiés, les cyclohexanes substitués, les carbonates d'alcools gras linéaires en C₆ à C₂₂, les carbonates de Guerbet, les esters de l'acide benzoïque avec des alcools linéaires et/ou ramifiés en C₆ à C₂₂, les dialkyléthers, les produits d'ouverture de cycle d'esters d'acides gras époxydés et de polyols, les huiles de silicone et les hydrocarbures aliphatiques ou naphténiques.

3. Procédé selon les revendications 1 et 2,
**caractérisé en ce qu'**
on utilise des alkyl- et/ou alcényl-oligoglycosides de formule (I),
R¹O-[G]ₚ (I)
dans laquelle R¹ représente un radical alkyle et/ou alcényle comportant de 4 à 22 atomes de carbone, G représente un radical saccharique comportant 5 ou 6 atomes de carbone et p représente des nombres allant de 1 à 10.

4. Procédé selon les revendications 1 à 3,
**caractérisé en ce qu'**
on utilise des éthersulfates d'alkyle.

5. Procédé selon les revendications 1 à 4,
**caractérisé en ce qu'**
on utilise des esters d'ammonium quaternaire de formule (II) 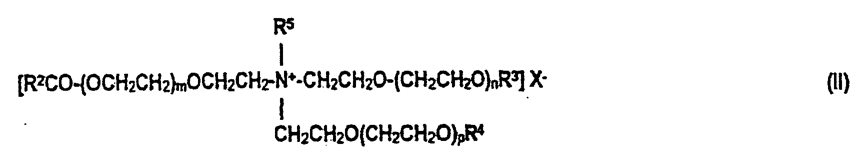 dans laquelle R²CO représente un radical acyle comportant de 6 à 22 atomes de carbone, R³ et R⁴ représentent indépendamment l'un de l'autre un hydrogène ou R²CO, R⁵ représente un radical alkyle comportant de 1 à 4 atomes de carbone ou un groupe (CH₂CH₂O)_{q}H , m, n et p valent au total 0 ou des nombres allant de 1 à 12, q représente des nombres allant de 1 à 12 et X représente un halogénure, un sulfate d'alkyle ou un phosphate d'alkyle.

6. Procédé selon les revendications 1 à 4,
**caractérisé en ce qu'**
on utilise des esters d'ammonium quaternaire de formule (III) 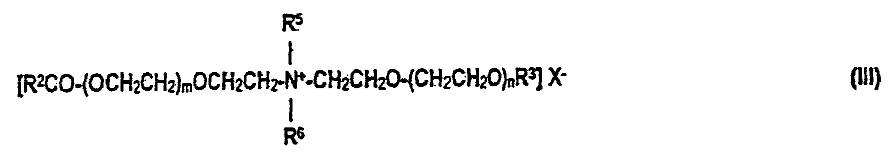 dans laquelle R²CO représente un radical acyle comportant de 6 à 22 atomes de carbone, R³ représente un hydrogène ou R²CO, R⁵ et R⁶ représentent indépendamment l'un de l'autre des radicaux alkyle comportant de 1 à 4 atomes de carbone, m et n valent au total 0 ou des nombres allant de 1 à 12 et X représente un halogénure, un sulfate d'alkyle ou un phosphate d'alkyle.

7. Procédé selon les revendications 1 à 4,
**caractérisé en ce qu'**
on utilise des esters d'ammonium quaternaire de formule (IV) 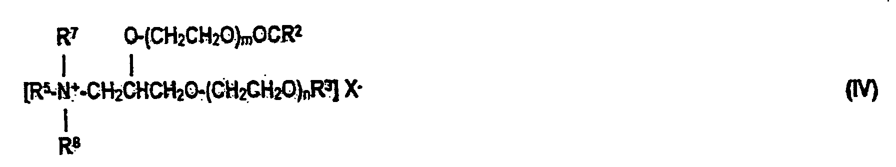 dans laquelle R²CO représente un radical acyle comportant de 6 à 22 atomes de carbone, R² représente un hydrogène ou R²CO, R⁵, R⁷ et R⁸ représentent indépendamment les uns des autres des radicaux alkyle comportant de 1 à 4 atomes de carbone, m et n valent au total 0 ou des nombres allant de 1 à 12 et X représente un halogénure, un sulfate d'alkyle ou un phosphate d'alkyle.

8. Procédé selon les revendications 1 à 7,
**caractérisé en ce que**
comme poly-12-hydroxystéarates de polyol on utilise des poly-12-hydroxystéarates de polyglycérine.

9. Procédé selon les revendications 1 à 8,
**caractérisé en ce qu'**
on utilise des alcools gras et/ou des polyéthylèneglycoléthers d'alcools gras de formule (V)
R⁹O(CH₂CH₂O)ₙH (V)
dans laquelle R⁹ représente un radical alkyle et/ou alcényle linéaire ou ramifié comportant de 12 à 22 atomes de carbone et n représente O ou des nombres allant de 1 à 25.

10. Procédé selon les revendications 1 à 9,
**caractérisé en ce qu'**
on ajoute aux émulsions des colorants agissant directement ou des colorants par oxydation.
